(19) Europäisches Patentamt
European Patent Office
Office européen des brevets

(11) Publication number: **0 461 261 A1**

(12) # EUROPEAN PATENT APPLICATION published in accordance with Art. 158(3) EPC

(21) Application number: **90907419.7**

(22) Date of filing: **10.05.90**

(86) International application number: **PCT/JP90/00598**

(87) International publication number: **WO 90/13228 (15.11.90 90/26)**

(51) Int. Cl.5: **A23L 1/305**, A23L 1/48, C12P 21/06

A request for addition of a third claim to priority has been filed pursuant to Rule 88 EPC. A decision on the request is pending.

(30) Priority: **12.05.89 JP 119960/89**
**28.02.90 JP 50415/90**

(43) Date of publication of application: **18.12.91 Bulletin 91/51**

(84) Designated Contracting States: **CH DE DK ES FR GB IT LI NL SE**

(71) Applicant: **OTSUKA PHARMACEUTICAL CO., LTD.**
**9, Kandatsukasacho 2-chome**
**Chiyoda-ku Tokyo 101(JP)**

Applicant: **FUJI OIL COMPANY, LIMITED**
**1-5, Nishishinsaibashi 2-chome Chuo-ku**
**Osaka-shi Osaka-fu(JP)**

(72) Inventor: **SATO, Kimihito**
**96-3, Aza Yawata Kurosaki Muya-cho**
**Naruto-shi**
**Tokushima 772(JP)**
Inventor: **SUGIYAMA, Kota**
**Saiwaicho Annex 602 101, Saiwaicho 3-chome**
**Tokushima-shi Tokushima 770(JP)**
Inventor: **MATSUBARA, Futoshi**
**3-10-24-508, Nagatoishi Kurume-shi**
**Fukuoka 830(JP)**
Inventor: **KIMOTO, Minoru**
**606 go, 2-5-2, Hagurazaki Izumisano-shi**
**Osaka 598(JP)**
Inventor: **MATSUO, Takaharu**
**973-34, Shindachiokanaka Sennan-shi**
**Osaka 590-05(JP)**
Inventor: **HASHIMOTO, Yukio**
**808-399, Higashigaokamachi Kishiwada-shi**
**Osaka 596(JP)**

(74) Representative: **Hansen, Bernd, Dr.rer.nat. et al**
**Hoffmann, Eitle & Partner Patentanwälte**
**Arabellastrasse 4 Postfach 81 04 20**
**W-8000 München 81(DE)**

(54) **OLIGOPEPTIDE MIXTURE AND COMPOSITION CONTAINING THE SAME.**

(57) An oligopeptide mixture prepared by the enzymatic hydrolysis of an animal or vegetable protein, wherein the average molecular weight is 200 to 5,000, the content of free amino acids is 35% by weight or less, the molar ratio of branched amino acids to aromatic amino acids is 7 or more, the content of branched amino acids in the total amino acids is 15 to 25% by weight, that of aromatic amino acids is 0.1 to 2.5% by weight, and that of methionine is 0.5 to 1.5% by weight; an alimentary composition containing the oligopeptide mixture for patients of hepatic diseases; and an alimentary or food composition containing the oligopeptide mixture.

EP 0 461 261 A1

## Field of the Invention

The present invention relates to an oligopeptide mixture prepared by the hydrolysis of a protein with enzymes, followed by removal of aromatic amino acid, a process for preparing the same, a nutritive composition for patients of liver diseases which contains the oligopeptide mixture and a nutritive or food composition containing the oligopeptide mixture.

## Background Art

Regarding cases of liver diseases, an imbalance of amino acid concentration is recognized in the plasma, namely the imbalance including the decrease of branched-chain amino acid (BCAA: valine, leucine, isoleucine), the increase of aromatic amino acid (AAA: phenylalanine, tyrosine), the decrease of BCAA/AAA molar ratio and the increase of methionine.

Now it is known that a supply of a protein source is effective for patients of liver diseases, the protein source being high in the BCAA content and low in the AAA and methionine contents and having a high BCAA/AAA molar ratio.

Thus amino acid preparations are known which are high in the BCAA/AAA molar ratio. However, these amino acid preparations suffer the drawbacks of, e.g. having a taste like that of pharmaceuticals, namely a bad taste, involving a high osmotic pressure and being expensive.

Oligopeptides, which are prepared by mere hydrolysis of a protein, do not have a satisfactorily high BCAA/AAA molar ratio. Oligopeptide preparations having a BCAA/AAA molar ratio increased by adding BCAA to the oligopeptide are known. However, since hydrolyzates of proteins are generally bitter, only the oligopeptides of less bitter taste prepared using a gelatin or collagen as a protein source are currently available for practical use.

Japanese Unexamined Patent Publication No.118839/1976 discloses an amino acid preparation for administration to patients of liver diseases. Japanese Unexamined Patent Publication No.11910/1982 describes a nutritive preparation for intrarectal administration, suitable for patients of liver diseases, the preparation containing a protein, a peptide and an amino acid in a specific ratio. Japanese Unexamined Patent Publication No.126767/1983 sets forth an elemental diet for patients of liver diseases. Japanese Unexamined Patent Publication No.500871/1987 discloses a dipeptide compound having a pharmacological activity. However, the peptide used in Japanese Unexamined Patent Publication No.11910/1982 is a mere protein hydrolyzate having a low BCAA/AAA molar ratio.

An oligopeptide mixture suited as a protein source for patients of liver diseases is unknown which is substantially free of bitterness, a high BCAA content and a high BCAA/AAA molar ratio, as found in the invention.

Recently protein hydrolyzates having a high content of peptide have been prepared by enzymolysis of an animal protein such as the white of an egg, casein or the like in an attempt to utilize the hydrolyzate, e.g. in supply of an artificial nutrient and in a diet therapy.

Japanese Unexamined Patent Publication No.287462/1988 proposes the use of an oligopeptide as a nutritive preparation, the oligopeptide being prepared by the hydrolysis of soybean protein with an enzyme to produce an oligopeptide having a peptide chain length of 2 to 10. The publication reports that the peptide is superior in the rapidly absorbing property to amino acids and is excellent as a fast-acting nutritive preparation useful in the marked consumption of physical strength caused by sports or the like.

Also proposed are various beverages for health containing amino acids, the beverages being prepared by chemically or enzymatically decomposing a protein. Especially Japanese Unexamined Patent Publication No.165774/1983 proposes a beverage composition (sports drink) containing BCAA. The publication states that BCAA has the effect of increasing the function of muscular motion, as supported by experimental results obtained by administering BCAA to rats to determine the quantity of motion.

However, the beverage composition disclosed in Japanese Unexamined Patent Publication No.165774/1983 necessitates the addition of BCAA which is prepared by chemical or enzymatical decomposition of a protein and selective separation of BCAA from the decomposition product, and entails a complicated procedure for separating BCAA, thereby posing the problem of high production costs. Further, amino acids have an additional problem that they generally taste strongly bitter and consequently impair the taste if added to a high concentration.

The peptide described in Japanese Unexamined Patent Publication No.287462/1988 have advantages of being higher in the rapidly absorbing property than amino acids and of not being so bitter as amino acids. The peptide, however, is not expected to provide the foregoing effect of giving a physiological activity by selective ingestion of a specific amino acid.

Disclosure of the Invention

It is an object of the present invention to provide an oligopeptide mixture suitable as a protein source or the like in a nutritive or therapeutic diet for patients of liver diseases or a nutritive preparation for use in consumption of physical strength, and a process for preparing the oligopeptide mixture.

It is another object of the invention to provide a nutritive composition for patients of liver diseases, the composition being capable of rectifying the amino acid pattern and the BCAA/AAA molar ratio in the plasma of patients of liver diseases, and ameliorating the nutriture thereof.

It is a further object of the invention to provide an oligopeptide-containing nutritive or food composition containing a large amount of an oligopeptide, the composition being rich in BCAA but low in the AAA content and having a good taste, the composition being suited to afford nourishment at the time of reduction of physical strength due to vigorous physical exercise, and being inexpensive to manufature on industrial basis.

The present inventors conducted research to resolve the aforesaid problems and made investigations of (1) the average molecular weight, (2) the BCAA/AAA molar ratio, (3) the BCAA content, (4) the AAA content, (5) the methionine content, (6) the free amino acid content, etc. which were obtained by changing the producing conditions as by hydrolyzing animal or plant proteins with an endopeptitase and an exopeptidase to liberate the AAA and absorbing the same with a synthetic adsorbent or activated charcoal for removal. As a result, the inventors found that a preparation for patients of liver diseases is efficacious in treating the patients, the preparation being produced using an oligopeptide mixture of the following composition as a protein source. The inventors also discovered that the amino acid pattern and BCAA/AAA ratio in the plasma of patients of liver diseases can be rectified and the nutriture can be ameliorated by utilizing the composition containing the oligopeptide mixture and that the composition is suitable for use in a nutritive or therapeutic diet for patients of liver diseases.

Another discovery by the inventors was that the composition containing the oligopeptide mixture of the following formulation is suited for use as a nutritive composition as in physical exercises or in consumption of physical strength.

The present invention has been accomplished based on these novel findings.

According to the invention, there are provided an oligopeptide mixture, a process for preparing the same and compositions containing the oligopeptide mixture, as described below:

1. an oligopeptide mixture prepared by the hydrolysis of an animal or plant protein with enzymes, the oligopeptide mixture being characterized in that the mixture has an average molecular weight of 200 to 5000, and contains not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7;

2. a process for preparing the oligopeptide mixture, the process being characterized by comprising the steps of subjecting an animal or plant protein to enzymolysis with an endoprotease and an exoprotease at neutrality range in the presence of an aqueous medium, treating the resulting decomposition product with an adsorbent, and when required, concentrating and/or drying the product;

3. a nutritive composition for patients of liver diseases, the composition containing an oligopeptide mixture prepared by the hydrolysis of an animal or plant protein with enzymes and the mixture having an average molecular weight of 200 to 5000 and containing not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7;

4. a nutritive composition for patients of liver diseases, the composition being characterized in that the composition contains 5 to 70% by weight, in terms of the dry weight, of an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7, and that the composition contains 10 to 90% by weight of total nitrogen compound as a protein source, 10 to 80% by weight of carbohydrate and 0 to 15% by weight of lipid in terms of the dry weight;

5. a nutritive or food composition which contains an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight of a branched-chain amino acid and 0.1 to 2.5% by weight of an aromatic amino acid, the (branched-chain amino acid/aromatic amino acid) molar ratio being not less than 7; and

6. a nutritive or food composition which contains an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7.

Suitably the oligopeptide mixture of the invention has an average molecular weight of 200 to 5000, preferably 200 to 3000. The oligopeptide mixture having an average molecular weight of less than 200 has the taste of an amino acid and entails a high osmotic pressure. The oligopeptide mixture having an average molecular weight of 200 to 5000 has the features that it can be rapidly digested and absorbed compared with proteins or amino acids and that it has a better savor than amino acids.

The oligopeptide mixture of the invention contains BCAA and AAA in a molar ratio of the former to the latter of not less than 7, preferably not less than 10, usually not more than 45 for practical use. Usually it is essential that preparations for patients of liver diseases have a BCAA/AAA molar ratio of not less than 5. The higher the value, the more desirable. Thus, commercially available preparations for patients of liver diseases usually have a BCAA/AAA molar ratio of not less than 20. Generally the higher the BCAA/AAA molar ratio, the higher the effect of increasing the function of muscular motion. While an oligopeptide prepared by mere enzymolysis of an animal or plant protein has a BCAA/AAA molar ratio of less than 7, the oligopeptide mixture of the invention is 7 or more in the BCAA/AAA molar ratio which is attributable to the treatment of the protein hydrolyzate with an adsorbent.

It is suitable that the oligopeptide mixture of the invention contain 15 to 25% by weight, preferably 17 to 25% by weight, of BCAA, based on the total amino acid composition. Patients of liver diseases have a reduced BCAA content in the blood, and thus a supply of BCAA to the patient is effective. Since the BCAA content is decreased after sports or after consumption of physical strength, a supply of BCAA is effective. The oligopeptide of the invention has the effect of supplying BCAA.

It is proper that the oligopeptide mixture of the invention contain 0.1 to 2.5% by weight, preferably 0.1 to 2.0% by weight, of AAA, based on the total amino acid composition. Since the BCAA content is decreased and the AAA content is increased in the blood of patients of liver diseases, the supply of AAA needs to be diminished. Similarly the BCAA content is decreased and the AAA content is increased in the blood affer sports and after consumption of physical strength, consequently necessitating the decrease in the supply of AAA. The oligopeptide mixture of the invention has an AAA content of as low as not more than 2.5% by weight.

Suitably the oligopeptide mixture of the invention contains 0.5 to 1.5% by weight, preferably 0.5 to 1.2% by weight, of a methionine, based on the total amino acid composition. Since the methionine content is increased in the blood of patients of liver diseases, the supply of methionine needs to be diminished. The oligopeptide mixture of the invention has a methionine content of as low as not more than 1.5% by weight.

The free amino acid content in the oligopeptide mixture of the invention is not more than 35% by weight, preferably not more than 30% by weight, more preferably not more than 15% by weight. The lower the free amino acid content, the more favorable. In the present invention, not more than 35% by weight of free amino acids are produced because (1) the enzymolysis is conducted by combined use of endoprotease and exoprotease to reduce markedly the bitterness, and (2) the treatment with an adsorbent is effected for adsorption and removal of AAA.

The oligopeptide mixture of the invention is prepared by the enzymolysis of a protein only at neutrality range (pH of 6 to 8), and therefore exhibits approximately neutrality. Thus without adjustment of pH, the oligopeptide mixture of the invention can be used itself as a protein source in a diet or in a preparation for patients of liver diseases.

The oligopeptide mixture of the invention is substantially free of bitterness and rather has a good taste and a pleasing flavor in contrast with conventional protein hydrolyzates which taste bitter. The reasons are that as described later, the oligopeptide mixture of the invention is prepared by causing the endoprotease and exoprotease to act on a protein at the same time, and the treatment with an adsorbent results in adsorption and removal of not only the AAA component but also ill-smelling components, savory components and coloring components.

Commercially available protein hydrolyzates have a strong bitterness and are difficult to orally administer even in case of use as a nutritive preparation, whereas the oligopeptide mixture of the invention has an advantage of being easy to orally administer.

The oligopeptide mixture of the invention is white in hue as compared with mere protein hydrolyzates.

The oligopeptide mixture of the invention as described above can be prepared by decomposing an animal or plant protein with an endoprotease and an exoprotease in the presence of an aqueous medium at neutrality range, treating the decomposition product with an adsorbent, and when required, concentrating and/or drying the product.

Among the animal or plant proteins for use as the starting material, useful animal proteins include, for example, casein, the white of an egg, albumin, collagen, gelatin, etc., and useful plant proteins include, for example, cereal proteins such as soybean protein, wheat protein, barley protein, azuki-bean protein, rye protein, corn protein, zein which belongs to prolamine-type proteins constituting a corn protein, seaweed proteins such as sea tangles, etc. Of these animal and plant proteins, plant proteins having a low content of methionine are suitable because the methionine content can be remarkably reduced by an enzymolysis and an adsorption procedure. While animal proteins of low methionine content are available, plant proteins are lower in the methionine content and higher in the BCAA content than animal proteins. Especially a soybean protein has a high BCAA content as well as a low methionine content and is industrially stably and easily available, hence suitable as a source of the oligopeptide mixture of the invention.

A suitable protein material is one which is made into an oligopeptide mixture by the process of the invention, the mixture having a methionine content of 0.5 to 1.5% by weight, (preferably 0.5 to 1.2% by weight), based on the total amino acid composition.

An endoprotease and an exoprotease are used as enzymes for the hydrolysis of proteins. Use of only endoprotease entails difficulty in releasing AAA or an oligopeptide rich in AAA, whereas use of only exoprotease fails to reduce the molecular weight of protein, and to obtain the desired oligopeptide mixture.

According to the invention, an oligopeptide mixture substantially free of bitterness can be produced by the combination of the simultaneous action of endoprotease and exoprotease and the below-mentioned treatment with an adsorbent. Commercially available decomposition products of proteins with enzymes are usually difficult to orally administer because of their bitterness, and thus are generally administered through a tube. With little or no bitterness, the oligopeptide mixture of the invention can be used as a protein source in preparations for patients of liver diseases or the like which are either administered through a tube or orally administered.

In the present invention, the endoprotease and the exoprotease to be used in combination may be of the same origin or different origins. Among them, combinations of such enzymes of the same origin are suitable. Among enzymes having both an endoprotease activity and an exoprotease activity, those of microorganism origin outnumber those of animal origin and those of plant origin. Proper examples of such enzymes of microorganism origin are those derived from molds (genus of Aspergillus, genus of Mucor, etc.), from bacteria (genus of Pseudomonas, etc.), from Actinomyces (genus of Streptomyces, etc.) or from yeasts (genus of Saccharomyces, etc.).

It is important in the invention that the enzymolysis be conducted only at neutrality range (usually pH of 6 to 8), regardless of an optimum pH of endoprotease or exoprotease, namely even in case of their optimum pH being in acidity or alkalinity range. This factor gives great economical advantages because the factor, for example, results in production of oligopeptides with a good flavor, and eliminates the need for an additional treatment for desalting, which is responsible for an increase of costs, with a reverse osmosis membrane, ion-exchange membrane or the like, or enables desalting with more inexpensive ion-exchange resins.

As to the degree of enzymolysis, the working temperature, the working time and the substrate/enzyme ratio are so adjusted that the solution of the oligopeptide mixture produced by the enzymolysis has an average molecular weight of 200 to 5000 (preferably 200 to 3000), and contains not more than 35% by weight (preferably not more than 15% by weight) of free amino acids.

Adsorbents suitable for use in the invention are those which are capable of specifically adsorbing AAA and preferably capable of also adsorbing a methionine. Usable as such adsorbents are activated charcoal, ion-exchange resins, hydrophobic adsorbing resins, etc. The adsorbent is used in an amount sufficient to adsorb AAA. For example, it is suitable to use commercially available activated charcoal in an amount in the range of from about 20% by weight, preferably about 50% by weight, to about 200% by weight, based on the amount of the crude protein after the enzymolysis. A lesser amount of the adsorbent used results in insufficient adsorption of AAA, whereas a larger amount of the adsorbent used leads to adsorption of large amounts of other amino acids and the oligopeptide, reducing the yield of the contemplated oligopeptide mixture.

The oligopeptide mixture solution obtained by removing the adsorbent by means of centrifugation, filtration or the like can be concentrated or dried as it is without neutralization and the ash content can be reduced usually to 15% by weight or less.

In this way, the oligopeptide mixture of the invention useful as a protein source or the like for patients of liver diseases can be obtained.

Use of only the adsorbent can not completely remove AAA, particularly tyrosine. The tyrosine may be separated and removed, as by isoelectric precipitation method, utilizing the low solubility of tyrosine, whereby the AAA content can be further reduced.

The oligopeptide mixture for use in the invention can be prepared by the above-metnioned procedures. When required, the liquid treated in the last step may be desalted, sterilized, concentrated or dried. Optionally the liquid may be treated with an enzyme(s) such as amidase, deaminase or the like before or after any of the above procedures in order to alleviate the bitterness.

The oligopeptide mixture of the invention can be used for an oral preparation for patients of liver diseases, or as a protein source which can be made into a commerically available preparation for patients of liver diseases (e.g. Japanese Unexamined Patent Publication No.11910/1982, etc.).

The nutritive composition for patients of liver diseases (hereinafter referred to as "composition for hepatopathy") according to the invention contains the foregoing oligopeptide mixture. Like conventional amino acid preparations, nitrogen compounds as a protein source, carbohydrates, lipids, vitamins, minerals and other additives when so required may be incorporated into the composition in addition to the oligopeptide mixture as the protein source, or may be administered together with the composition of the invention. The incorporation or simultaneous administration of the additional compounds can retain the nutrition balance in the patients of liver diseases.

Nitrogen compounds useful as the protein source can be any of conventional protein materials, amino acids, etc. Examples of useful protein materials are casein, salts of caseins such as sodium caseinate and calcium caseinate, decomposition products of these casein products with enzymes, soybean protein, decomposition products of wheat protein with enzymes, etc. The nitrogen compound can be used in an amount corresponding to up to 5 times the weight of the oligopeptide mixture.

Useful carbohydrates can be any of conventional ones, and include, for example, glucose, maltose, sucrose, isomaltose, maltotetraose, maltotriose, maltopentaose, maltohexaose, lactose, glycogen, dextrin, starch and like monosaccharides, oligosaccharides, dietary fibers, polysaccharides, etc. The carbohydrate can be added usually in an amount corresponding to up to about 10 times the total weight of the protein source.

Useful lipids can be any of conventional lipids including animal and plant oils, such as rice oil, cottonseed oil, corn oil, soybean oil, sunflower oil, cacao fat and oil, sesame oil, safflower oil, peanut oil, butter, lard, coconut oil, nut oil, palm oil, rapeseed oil, medium-chain triglyceride (MCT), etc. among which plant oils are preferred. The lipid can be used usually in an amount corresponding to up to about twice the total weight of the protein source.

Examples of useful vitamins are vitamin A, vitamin $B_1$, vitamin $B_2$, vitamin $B_6$, vitamin $B_{12}$, vitamin C, vitamin D, vitamin E, niacin, folic acid, pantothenic acid, etc. Examples of useful minerals are salts of calcium, iron, potassium, sodium, magnesium, phosphorus, chlorine, etc. It is suitable to use the vitamin in an amount corresponding to up to about 0.01 times, and the mineral in an amount corresponding to up to about 0.05 times, the total weight of the protein source. Other additives useful in the invention include, for example, synthetic flavour, natural flavour and like flavours, natural sweetener (thaumatin, stevioside), synthetic sweetener (saccharin, aspartame) and like sweeteners, coloring agents, emulsifiers, stabilizers, antiseptics, etc. These additives are usable singly, or at least two of them can be used in mixture.

Suitably the composition of the invention for hepatopathy contains about 5% by weight or more of the oligopeptide mixture in terms of the dry weight.

The composition containing the oiligopeptide mixture of the invention in the below-specified amount and having total nitrogen compound as a protein source, carbohydrate and lipid in the below-specified amounts is useful as a composition for hepatopathy which is well balanced in the nutrition. The amounts of the components in the composition for hepatopathy are as follows.

The amount of the oiligopeptide mixture used is 5 to 70% by weight, preferably 5 to 20% by weight, based on the dry weight of the composition for hepatopathy. As to the respective amounts of total nitrogen compound as a protein source, carbohydrate and lipid, the composition contains 10 to 90% by weight of the nitrogen compound, 10 to 80% by weight of the carbohydrate and 0 to 15% by weight of the lipid, preferably 15 to 40% by weight of the nitrogen compound, 50 to 70% by weight of the carbohydrate and 3 to 10% by weight of the lipid, based on the dry weight of the composition. The respective amounts of the nitrogen compound, carbohydrate and lipid in the composition for hepatopathy in which the amounts of the nitrogen compound, carbohydrate and lipid are specified are the respective combined amounts of these components to be contained in the oiligopeptide mixture, and the components to be added.

The inclusion of the nitrogen compound, carbohydrate and lipid at the above-specified ratio in the composition for hepatopathy serves to rectify the balance of amino acids in the blood and to improve the nutriture.

The nitrogen compounds as a protein source constituting the composition for hepatopathy which contains specified amounts of the nitrogen compound, carbohydrate and lipid can be any of conventional protein materials, amino acids and the like, insofar as the above-specified oligopeptide mixture is contained in the above-defined amount. Useful protein materials include those exemplified above. Preferred amino acids are valine, leucine, isoleucine and like branched-chain amino acids. These nitrogen compounds are usable singly, or at least two of them can be used in mixture.

The carbohydrate may be present in the composition for hepatopathy in addition to that contained in the oligopeptide mixture can be any of conventional ones, such as those exemplified above.

The lipid may be present in the composition for hepatopathy in addition to that contained in the oligopeptide mixture may be selected from various conventional ones including the examples thereof shown above.

The composition for hepatopathy comprising specific amounts of the components may contain additives commonly employed when so required in addition to the foregoing three essential components. Examples of useful additives include, for example, the above-exemplified vitamins, minerals, flavours, sweeteners, coloring agents, emulsifiers, stabilizers, anticeptics, etc. These additives are usable singly, or at least two of them can be used in mixture.

The composition of the invention for hepatopathy which is an intimate mixture of powdery components is hermetically enclosed in moisture-proof bags, bottles, cans or the like, for storage or transport. When desired, the composition may be formulated into, e.g. solutions, jellies, granules, tablets, capsules, etc.

The thus obtained composition of the invention for hepatopathy is diluted usually with water into a solution or a jelly having a proper concentration in a conventional manner before administration. The solution or the jelly may be sterilized by heating or by heating under pressure. The degree of the dilution can be determined as desired. In the preparation of a nutritive liquid for oral administration or administration through a tube, usually it is suitable to dilute the composition of the invention so that the obtained nutritive liquid contains about 100 to about 500 g/l, preferably about 150 to about 250 g/l, of the composition of the invention. The foregoing nutritive liquid is orally administered or administered through a tube directly into the stomach, duodenum or small intestine. The preparation in the form of a jelly or the like can be orally administered.

The dose of the composition of the invention for hepatopathy is suitably determinable depending on the symptom of a patient to be applied therewith, the intended effect of therapy or nutrition improvement, etc. A desirable daily dose is generally about 10 to about 60 g per patient, calculated as the oligopeptide mixture in the composition for hepatopathy. In case of the composition for hepatopathy containing specific amounts of the nitrogen compound, carbhydrate and lipid, about 30 to about 150 g of the composition in terms of the dry weight is preferably administered daily for one patient.

The composition of the invention for hepatopathy may be taken together with a usual meal according to the patient's liking.

It is possible to use the composition of the invention as a food or nutritive one (hereinafter referred to as a nutritive preparation") at the time of exercise or consumption of physical strength, the composition containing an oligopeptide mixture prepared by the hydrolysis of a plant protein with enzymes, the mixture having an average molecular weight of 200 to 5000, preferably 200 to 3000, the mixture containing 35% by weight or less, preferably 30% by weight or less, of free amino acids and, based on the total amino acid composition, 15 to 20% by weight, preferably 17 to 25% by weight, of BCAA, and 0.1 to 2.5% by weight, preferably 0.1 to 2.0% by weight, of AAA, the BCAA/AAAA molar ratio being not less than 7, preferably not less than 10.

The foregoing oligopeptide mixture contained in the nutritive preparation can be obtained, for example, by the above-mentioned process of the invention for preparing the oligopeptide mixture.

When a methionine is contained in the oligopeptide mixture to be incorporated into the nutritive preparation of the invention, suitably the methionine is in a low concentration because it emits an offensive odor. It is proper that the methionene content be preferably 0.5 to 1.5% by weight, more preferably 0.5 to 1.2% by weight, based on the total amino acid composition.

The nutritive preparation of the invention essentially contains the oligopeptide mixture having the foregoing formulation and is used in the same form as that of conventional nutritive or food compositions, such as beverages, granules, powders, effervescent preparations, etc.

Described below are preferred modes of processes for producing the nutritive preparations of the invention in the form of beverages, granules, powders, tablets and effervescent preparations.

The beverage of the invention can be prepared by dissolving the oligopeptide mixture into water. In this case, the amount of the oligopeptide mixture as dissolved is preferably 0.5 g/l or more. A lesser content of the oligopeptide mixture results in failure to obtain a satisfactory effect of the invention. The beverage may contain, in addition to the oligopeptide mixture, at least one additive selected from the group consisting of sweeteners such as sugar, fructose, glucose, etc.; sourness-imparting agents such as citric acid, sodium citrate, tartaric acid, lactic acid, fumaric acid, malic acid, etc.; vitamins such as vitamin A, vitamin $B_1$, vitamin $B_2$, vitamin $B_6$, vitamin $B_{12}$, vitamin C, vitamin $D_3$, vitamin E, vitamin $K_3$, vitamin D, biotin, folic acid, calcium pantothenate, paraaminoaromatic acid, nicotinic acid, etc.; minerals such as calcium phosphate, potassium phosphate, sodium phosphate, sodium chloride, calcium lactate, iron citrate, magnesium sulfate, zinc carbonate, manganese sulfate, copper sulfate, potassium iodide, etc.; carbonic acid; alcohols such as ethanol, propanol, etc.; flavours; etc.

The granules of the invention can be obtained by mixing the oligopeptide mixture with other components when so required and granulating the mixture into granules of suitable diameter as by centrifugal fluidization-type coating granulation method, fluidized bed coating granulation method, spray coating granulation method using a coating pan or the like, followed, when required, by drying the granules. The powder of the invention can be prepared by making the oligopeptide mixture into powders, mixing the particles with other components when so required and dividedly wrapping up the mixture in a proper quantity. The tablets of the invention can be prepared by mixing the oligopeptide mixture with other components when so required and shaping the mixture as by direct powder compression method, dry- or wet-type granule compression method or the like.

Freely usable as the other components in the above production processes is at least one component selected from the group consisting of: excipients such as lactose, sugar, sodium chloride, glucose, starch, calcium carbonate, kaolin, crystalline cellulose, silicate, etc.; binders such as water, ethanol, propanol, methylene chloride, simple syrup, glucose solution, starch solution, gelatin solution, carboxymethyl cellulose Na, shellac, methyl cellulose, hydroxypropyl methyl cellulsoe, hydroxypropyl cellulose, polyvinyl pyrrolidone, polyvinyl alcohol, etc.; disintegrators such as dried starch, agar powder, carboxymethyl cellulose calcium, sodium hydrogencarbonate, calcium carbonate, polyoxyethylene sorbitan fatty acid ester, sodium lauryl sulfate, monoglyceride stearate, starch, etc.; absorption accelerators such as quaternary ammonium base, sodium lauryl sulfate, etc.; lubricants such as purified talc, stearate, boric acid powder, polyethylene glycol, colloidal silica, etc.; plasticizers such as glycerin fatty acid ester, dioctylphthalate, dibutylphthalate, triacetin, castor oil, etc.; the above sweeteners, sourness-imparting agents, vitamins, minerals, flavours, etc.

The effervescent preparation of the invention can be prepared by mixing the oligopeptide mixture with sodium hydrogencarbonate and/or sodium carbonate and a neutralizing agent as essential components, and other components when so required, and making the mixture into granules, powders or tablets. The term "neutralizing agent" used herein refers to acidic compounds which are capable of neutralizing the sodium hydrogencarbonate and/or sodium carbonate to produce carbon dioxide gas. Preferred examples of such neutralizing agents are organic acids such as L-tartaric acid, citric acid, lactic acid, malic acid, fumaric acid, ascorbic acid, etc. As to the proportions of the components in the effervescent preparation, it is desirable to use 5 to 40% by weight of the sodium hydrogencarbonate and/or sodium carbonate and 5 to 40% by weight of the neutralizing agent. Freely added as other components is at least one component selected from the group consisting of the above-mentioned excipients, binders, disintegrators, absorption accelerators, lubricants, plasticizers, sweeteners, sourness-imparting agents, vitamins, minerals, flavours, etc. This effervescent preparation is foamed on immersion into water, and rapidly dissolved therein, and thus the solution can be taken as a beverage.

The amount of the oligopeptide mixture contained in the nutritive or food composition of the invention is preferably at least 10% by weight. If a less amount of the mixture is used, the composition is needed in a larger amount to obtain the effect of the invention.

As described above, the present invention provides the oligopeptide mixture as a protein source which is suitable for patients of liver diseases and which has a good flavor. The oligopeptide mixture can be widely used for oral or tube-route administration as a protein source in preparations for patients of liver diseases or as a protein source in a diet for such patients.

Further, the composition of the invention for hepatopathy contains chiefly the oligopeptide mixture as a protein source, has a good flavor free of offensive bitterness, and can be rapidly and properly absorbed through a digestive tract with unliklihood to cause diarrhea due to a high osmotic pressure. And the composition of the invention can significantly improve the amino acid pattern and the BCAA/AAA molar ratio (Fischer's ratio) in the plasma of a patient of hepatopathy. Furthermore, the composition of the invention having incorporated therein a well-balanced combination of a protein source, carbohydrate and fat can ameliorate the nutriture. Because of these factors, the composition of the invention is suitable for use as a therapeutic preparation or a nutritive preparation for acute hepatitis, convalescence of hepatitis, chronic hepatitis, and compensated or decompensated hepatocirrhosis, particularly as a nutritive or therapeutic preparation for patients of acute or chronic hepatic insufficiency and like liver diseases.

The nutritive preparation of the invention contains the oligopeptide mixture with a molecular weight of 200 to 5000 prepared by the hydrolysis of a protein with enzymes. Like amino acids, the oligopeptide is rapidly absorbed well through a digestive tract and involves a low osmotic pressure so that its relatively great intake does not cause diarrhea, hence is suitable for supply of a nutrient at the time the physical strength has been lowered as by vigorous exercise.

The oligopeptide mixture contains AAA in a ratio of 2.5% by weight or less based on the total amino acid composition and is rich in BCAA so that the mixture is expected to achieve effects such as inhibition of decomposition of myoprotein at the time of exercise, promotion of synthesis of myroprotein and increase of function of muscular motion.

Generally peptides have a less bitter taste than amino acids. In the invention, since the bitterness is further diminished by reduction of the AAA content, improvements are achieved in the odor, taste and the like, so that a product excellent in flavor and taste is provided.

Consequently the nutritive preparation of the invention is suited for use as a nutritive preparation as in exercise, fatigue, medical treatment of diseases, therapy of malnutrition due to origotrophy or apastia, and is expected to produce high efficacies in the increase of physical strength, recovery from fatigue, etc.

Given below are Examples to clarify the features of the invention in greater detail with reference to the accompanying drawings.

Fig. 1 shows the distribution of the molecular weight of the oligopeptide mixture obtained in Example 1, as determined by a high performance liquid chromatography; Fig. 2 is a graph showing the change of the amino acid concentration with time in the blood in Test Example I; Fig. 3 is a graph showing the change of the free amono acid concentration with time in the muscular tissue in Test Example I; Fig. 4 is a graph showing the change of the BCAA concentration with time in Test Example II; Fig. 5 is a graph showing the change of the BCAA/AAA ratio with time in Test Example II; and Fig. 6 is a graph showing the results of measurement of the free amino acid concentration in the plasma and the ammonia concentration in the blood in Test Example III.

Examples

Example 1

A 100 parts by weight (hereinafter simply referred to as "parts") quantity of isolated soybean protein (trade name: New Fujipro R, product of Fuji Oil Co., Ltd.) was dissolved in 1567 parts of water. To the solution was added 2 parts of a protease (trade name: Pronase, product of Kaken Pharmaceutical Co., Ltd.) containing endopeptidase and exopeptidase. The resulting mixture was subjected to enzymolysis at 50°C for 5 hours, heated at 80 to 85°C for 15 minutes for inactivation of the enzymes and cooled to not higher than 10°C. A 100 parts quantity of activated charcoal (product of Takeda Chemical Industries, Ltd.) was added thereto and the mixture was stirred overnight to cause aromatic amino acid and an oligopeptide having an abundance of aromatic amino acid to adsorb on the activated charcoal. The resulting mixture was centrifuged (at 5000 r.p.m., for 30 minutes) to roughly remove the activated charcoal and then the activated charcoal not removed was completely removed with use of a precision filtration membrane (product of Asahi Chemical Industry Co., Ltd., 0.1 $\mu$). The resulting filtrate was sterilized (at 135°C for 7 seconds) and subjected to spray drying, giving a particulate oligopeptide mixture useful for treating patients of liver disease. The 10 wt.% aqueous solution of the mixture obtained above had a pH of 6.3.

Tables 1 and 2 show the compositions of the oligopeptide mixture before the adsorption on the activated charcoal (HSP) and the oligopeptide mixture after such adsorption (LAP), both calculated as dry solids, in terms of the proportions of crude protein, ash and amino acids.

Table 1

| | HSP | LAP |
|---|---|---|
| Crude protein | 83.5 wt.% | 81.0 wt.% |
| Ash | 6.3 wt.% | 10.5 wt.% |

The amino acids in Table 2 are designated according to the regulations of IUPAC and IUB or by the symbols commonly used in the art. Examples of such designation are shown below. In the case where the amino acids or the like have optical isomers, such isomers are all those of L-type unless otherwise specified.

Val: valine; Leu: leucine; Ile: isoleucine; Ala: alanine; Glu: glutamic acid; Thr: threonine; His: histidine; Ser: serine; Gly: glycine; Asn: asparagine; Arg: arginine; Asp: aspartic acid; Pro: proline; Tyr: tyrosine; Phe: phenylalanine; Cys: cystine; Met: methionine; Lys: lysine; Trp: tryptophan

Table 2

| Amino acid | HSP (wt.%) | LAP (wt.%) |
|---|---|---|
| Tyr | 3.1 | 0.7 |
| Phe | 5.4 | 1.3 |
| Thr | 3.8 | 4.4 |
| Cys | 1.2 | 1.1 |
| Met | 1.3 | 0.9 |
| Val | 4.7 | 5.3 |
| Ile | 4.8 | 4.9 |
| Leu | 7.9 | 9.1 |
| Lys | 6.2 | 6.9 |
| Trp | 1.5 | 0 |
| His | 2.2 | 1.9 |
| Asp | 11.6 | 13.6 |
| Ser | 5.3 | 5.5 |
| Glu | 19.4 | 23.9 |
| Pro | 4.7 | 5.2 |
| Gly | 4.1 | 4.1 |
| Ala | 4.2 | 4.6 |
| Arg | 8.7 | 6.6 |

From the data as indicated above in Tables 1 and 2, it was revealed that LAP was found to contain aromatic amino acids (AAA: phenylalanine, tyrosine), branched-chain amino acids (BCAA: valine, leucine, isoleucine) and free amino acids (FAA) in the proportions as shown below in Table 3 and tee molar ratio of BCAA/AAA in LAP was also shown in Table 3. In Table 3, the quantities of AAA, BCAA and methionine are determined based on the total amino acid composition.

Table 3

| | HSP | LAP |
|---|---|---|
| AAA | 8.5 wt.% | 2.0 wt.% |
| BCAA | 17.5 wt.% | 19.3 wt.% |
| Molar ratio of BCAA/AAA | 2.75 | 12.9 |
| Methionine | 1.3 wt.% | 0.9 wt.% |
| FAA | 18 wt.% | 25 wt.% |

The molecular-weight distribution of LAP as determined by high performance liquid chromatogaphy was shown in Fig. 1. The molecular-weight distribution was determined under the following conditions: the column used: TSKgel G3000 PW (17.8 mmØ x 30 cm); eluent: 0.1% trifluoroacetic acid containing 45% acetonitrile; flow rate: 0.3 ml/min. The detection was conducted using UV of 220 nm and the molecular-weight distribution was expressed in terms of absorbance.

The LAP obtained above was free of bitter taste and had a slight flavor, hence desirable.

Example 2

An oligopeptide mixture was prepared in the same manner as in Example 1 with the exception of using, as shown below in Table 4, "AOS (type II)" (trade name, product of Seishin Pharmaceutical Co., Ltd.) or "Protease M" (trade name, product of Amano Pharmaceutical Co., Ltd.), each originating in Aspergillus oryzae and containing endoprotease and exoprotease at the same time. In Table 4, the quantities of AAA, BCAA and methionine were determined based on the total amino acid composition.

Table 4

| Name of enzyme | AOS (Type II) | Protease M |
|---|---|---|
| Quantity used | 4 wt.% | 3 wt.% |
| Average molecular weight | 390 | 340 |
| AAA | 1.9 wt.% | 1.7 wt.% |
| BCAA | 18.5 wt.% | 19.5 wt.% |
| Molar ratio of BCAA/AAA | 13.0 | 15.3 |
| Methionine | 1.1 wt.% | 0.9 wt.% |
| FAA | 22.3 wt.% | 24.0 wt.% |

Comparison Example 1

The same procedure as in Example 2 was repeated with the exception of using "Protine AC" (trade name, product of Amano Phamaceutical Co., Ltd.) originating in Bacillus Subtilus and containing only endoprotease as a protease, giving an oligopeptide mixture.

Table 5 shows the results. The quantities of AAA, BCAA and methionine were determined based on the total amino acid composition.

Table 5

| Name of enzyme | Protine AC |
|---|---|
| Quantity used | 4 wt.% |
| Average molecular weight | 850 |
| AAA | 3.5 wt.% |
| BCAA | 17.9 wt.% |
| Molar ratio of BCAA/AAA | 6.87 |
| Methionine | 1.1 wt.% |
| FAA | 3.8 wt.% |

It was revealed that the mere use of endoprotease encountered difficulty in the liberation of AAA.

Example 3

The same procedure as in Example 1 was repeated with the exception of changing the quantity of the activated charcoal used as shown in Table 6, giving an oilgopeptide mixture. Table 6 also shows the results. In Table 6, the quantity of AAA was determined based on the total amino acid composition.

Table 6

| Activated charcoal/soybean protein | AAA | Molar ratio of BCAA/AAA |
|---|---|---|
| 10 wt.% | 3.0 | 6.0 |
| 20 wt.% | 2.5 | 7.5 |
| 100 wt.% | 1.9 | 10.0 |
| 200 wt.% | 1.5 | 13.8 |

From the results shown in Table 6, it was revealed that a suitable quantity of the activated charcoal was 20 wt.% or more based on the amount of soybean protein.

Example 4

A 100 parts quantity of isolated soybean protein, "New Fujipro R" (appearing hereinbefore), was dissolved in 900 parts of water, and 4 parts of Protease-M (appearing hereinbefore) was added to the solution. The resulting mixture was subjected to enzymolysis at 50°C for 5 hours and thereafter heated at 80 to 85°C for 15 minutes to inactivate the enzymes, followed by cooling to up to 10°C. Then 110 parts of activated charcoal (product of Takeda Chemical Industries, Ltd.) was added thereto and the mixture was stirred overnight, thereby causing the adsorption of AAA and an oligopeptide containing an abundance of AAA on the activated charcoal. The activated charcoal was filtered off and an ion exchange resin (a mixture of "Diaion SK-116" manufactured by Mitsubishi Chemical Industries Limited and "KA 890" manufactured by Sumitomo Chemical Company, Limited in a ratio by weight of 1:1) was added to the filtrate. The mixture was stirred for 1 hour and the resin was filtered off, giving a desalted mixture. The desalted mixture (pH: 5.2) was subjected to precision filtration and then to spray drying. The dried product had a slight acid taste but was free of bitter or unplesant taste, hence desirable. Table 7 shows the composition and the like of the product. In Table 7, the quantities of AAA, BCAA and methionine were determined based on the total amino acid composition.

Table 7

| | |
|---|---|
| Crude protein | 80.4 wt.% |
| FAA | 24.8 wt.% |
| Ash | 3.6 wt.% |
| AAA | 0.9 wt.% |
| BCAA | 20.7 wt.% |
| Methionine | 0.9 wt.% |
| BCAA/AAA | 28.7 (molar ratio) |

Example 5

The same procedure as in Example 1 was repeated except that 100 parts of lactalbumin (trade name: "Sunlact N-2", product of Taiyo Chemical Co., Ltd.) was dissolved in 1667 parts of water, giving an oligopeptide mixture. Table 8 shows the results. In Table 8, the quantities of AAA, BCAA and methionine were determined based on the total amino acid composition.

Table 8

| | |
|---|---|
| Crude protein | 73.1 wt.% |
| FAA | 19.8 wt.% |
| Ash | 4.6 wt.% |
| AAA | 0.9 wt.% |
| BCAA | 23.8 wt.% |
| Methionine | 1.0 wt.% |
| BCAA/AAA | 33.0 (molar ratio) |

Example 6

Using the oligopeptide mixtures obtained in Examples 1 to 5, a nutritive composition for patients of liver disease according to the present invention was prepared by mixing the components used so that the nutritive composition had the composition (expressed in gram) as shown below in Table 9 based on 100 g of the composition on dry basis.

Table 9

| | | 1 | 2 | 3 | 4 | 5 |
|---|---|---|---|---|---|---|
| Nitrogen compound | Oligopeptide mixture | Example 1 15 | Example 2 13.5 | Example 3 18.0 | Example 4 15.0 | Example 5 15.0 |
| | Soybean peptide | | 13.5 | 9.0 | | |
| | Casein | 8 | | | 20.0 | 15.0 |
| | Gelatin | | | | | |
| | Tryptophan | | Proper qt. | Proper qt. | | |
| | Taurine | 3 | | | | |
| | Valine | | | | | |
| | Leucine | | | | | |
| | Isoleucine | | | | | |
| Carbo-hydrate | Rice dextrin | 55 | 62.1 | 62.1 | 40 | 60 |
| | Dietary fibre | | | | 10 | |
| | Oligosaccharide | 5 | | | 10 | |
| | Purified saccharose | | | | | |
| Lipid | Rice (bran) oil | 5 | 7.0 | 7.0 | 3.0 | 4.0 |
| | Medium-chain triglyceride | 4 | | | | 3.0 |
| Others | Vitamin, Mineral | Proper qt. | Proper qt. | Proper qt. | Proper qt. | Proper qt. |

Table 9 (continued)

| | | 6 | 7 | 8 | 9 | 10 |
|---|---|---|---|---|---|---|
| Nitrogen compound | Oligopeptide mixture | Example 1 18 | Example 2 15 | Example 3 12 | Example 4 20 | Example 5 10 |
| | Soybean peptide | | | 3 | 5 | |
| | Casein | 8 | 6 | 8 | 5 | 10 |
| | Gelatin | | 4 | | | 5 |
| | Tryptophan | | | | | |
| | Taurine | 3 | 3 | 3 | 3 | 3 |
| | Valine | | | | 0.1 | 0.5 |
| | Leucine | | | | 0.1 | 0.5 |
| | Isoleucine | | | | 0.1 | 0.5 |
| Carbo-hydrate | Rice dextrin | 45 | 50 | 45 | 45 | 50 |
| | Dietary fibre | 5 | 5 | 5 | 5 | 5 |
| | Oligosaccharide | 5 | 5 | 5 | 5 | 3 |
| | Purified saccharose | | | 5 | | |
| Lipid | Rice (bran) oil | 3 | 4 | 6 | 2 | 3 |
| | Medium-chain triglyceride | 3 | 4 | 4 | 2 | 3 |
| Others | Vitamin, Mineral | Proper qt. | Proper qt. | Proper qt. | Proper qt. | Proper qt. |

Examples 7 to 9 (Drinks)

Drinks were produced by dissolving the components as listed below in Table 10 in 1 ℓ of water. The oligopeptide mixture used was that obtained in Example 1.

The drinks obtained in Examples 7 to 9 were each given to ten subjects. The subjects evaluated the drinks with the result that all the three drinks were palatable and tasted good. After the drinking, no one was suffered from diarrhea or like unpleasant symptom.

Table 10

| Components | Example 7 | Example 8 | Example 9 |
|---|---|---|---|
| Oligopeptide mixture | 25 g | 50 g | 100 g |
| Purified saccharose | 70 g | " | " |
| Rice (bran) oil | 42 g | " | " |
| Vitamin A | 11500IU | " | " |
| Vitamin $B_1$ | 9.2 mg | " | " |
| Vitamin $B_2$ | 9.2 mg | " | " |
| Vitamin $B_6$ | 9.2 mg | " | " |
| Vitamin $B_{12}$ | 27.7 $\mu$g | " | " |
| Vitamin C | 3346.4 mg | " | " |
| Vitamin D | 9923.6IU | " | " |
| Vitamin E | 69.3IU | " | " |
| Pantothenic acid | 46.2 mg | " | " |
| Niacin | 92.4 mg | " | " |
| Folic acid | 1847.2 $\mu$g | " | " |
| Biotin | 1385.4 $\mu$g | " | " |
| Vitamin K | 692.7 $\mu$g | " | " |
| Choline | 1154.5 mg | " | " |
| Ca | 2309.0 mg | " | " |
| $PO_4$ | 2309.0 mg | " | " |
| Mg | 923.6 mg | " | " |
| Na | 3232.6 mg | " | " |
| K | 6003.4 mg | " | " |
| Cl | 4618.0 mg | " | " |
| Fe | 73.9 mg | " | " |
| Zn | 36.9 mg | " | " |
| Cu | 4.6 mg | " | " |
| Mn | 92.4 mg | " | " |
| I | 346.4 $\mu$g | " | " |
| Flavor | Proper qt. | " | " |

<u>Examples 10 to 13 (Drinks)</u>

Drinks were produced by dissolving the components as listed below in Table 11 in 1 ℓ of water. The oligopeptide mixture used was that obtained in Example 2.

Table 11

| Components (g) | Example | | | |
|---|---|---|---|---|
| | 10 | 11 | 12 | 13 |
| Oligopeptide mixture | 25 | 50 | 100 | 200 |
| Stevioside | 3 | 3 | 3 | 3 |
| Ascorbic acid | 10 | 10 | 10 | 10 |

The thus obtained drinks of Examples 10 to 13 were all palatable and tasted good.

Example 14 (Effervescent tablets)

The components as listed in Table 12 were mixed together and the mixture was directly subjected to a powder-compression method to prepare tablets. The obtained tablets were effervescent tablets each weighing 10 g. The oligopeptide mixture used was that prepared in Example 3.

Table 12

| Components | Amount (% by weight) |
|---|---|
| Oligopeptide mixture | 50 |
| L-Ascorbic acid | 7 |
| Aspartame | 0.5 |
| L-Tartaric acid | 10 |
| Sodium hydrogen carbonate | 12 |
| Granulated sugar | 18 |
| Cyanocobalamin | Small qt. |
| Flavor and pigment | Small qt. |

When one of the effervescent tablets thus obtained was immersed in 180 ml of water, the tablet effervesced and rapidly dissolved to form a homogeneous solution. The solution was palatable and tasted good.

Examples 15 to 21 (Effervescent tablets)

The components as shown below in Table 13 were mixed together and the mixture was directly subjected to a powder-compression method, giving effervescent tablets. The oligopeptide mixture used was that produced in Example 4.

When each tablet thus obtained was immersed in 180 ml of water, the tablet effervesced and rapidly dissolved to form a homogeneous solution. Each solution thus obtained was palatable and tasted good.

Examples 22 to 26 (Effervescent powders)

The components as shown below in Table 14 were weighed and mixed together in the predetermined amounts. When required, 0 to 5% by weight of a sweetener and 0 to 1% by weight of a flavor were further added to the mixture obtained above. The mixture obtained was divided into wrappers each containing a suitable quantity (as shown in Table 14) of the mixture, giving effervescent powders. The oligopeptide mixture used was that produced in Example 1.

When each wrapper of the effervescent powder was immersed in 180 ml of water, the powders effervesced and rapidly dissolved, giving homogeneous solutions. The obtained solutions were all palatable and tasted good.

Table 13

| Components (part) | Example | | | | | | |
|---|---|---|---|---|---|---|---|
| | 15 | 16 | 17 | 18 | 19 | 20 | 21 |
| Oligopeptide mixture | 45 | 50 | 60 | 70 | 50 | 60 | 80 |
| Granulated sugar | 18 | 17 | 10 | 5 | 6 | - | - |
| L-Ascorbic acid | 6 | 3 | 5 | - | 10 | - | - |
| L-Tartaric acid | 11 | 11 | 10 | 10 | 12 | 15 | 9 |
| Aspartame | 0.6 | 0.8 | 1.0 | 1.2 | 1.0 | 3.0 | 2.0 |
| Sodium hydrogen carbonate | 17 | 16 | 10 | 10 | 17 | 20 | 9 |
| Cyanocobalamin | Small qt. | Small qt. | Small qt. | Small qt. | Small qt. | Small qt. | Small qt. |
| Flavor and pigment | Proper qt. | Proper qt. | Proper qt. | Proper qt. | Proper qt. | Proper qt. | Proper qt. |
| Weight of preparation (g/tablet) | 5.0 | 7.5 | 10.0 | 5.0 | 2.5 | 2.5 | 5.0 |

Table 14

| Component (g) | Example | | | | |
|---|---|---|---|---|---|
| | 22 | 23 | 24 | 25 | 26 |
| Oligopeptide mixture | 5 | 8 | 4 | 6 | 5 |
| L-Tartaric acid | 2 | 0.8 | 0.4 | 1 | 0.8 |
| Sodium hydrogen carbonate | 3 | 1.2 | 0.6 | 2 | 2 |
| Weight of preparation (g/wrapper) | 10 | 10 | 5 | 9 | 7.8 |

Test Example I

The oligopeptide mixture used was the same as that prepared from isolated soybean protein in the same manner as in Example 1. The amino acid composition and the quantities of other components used were shown below in Table 15.

Six-week old male Sprague-Dawley rats (38 rats, weighed 213.0 ± 9.2 g (average ± standard deviation)) were each caused to fast for 18 hours, and the test sample was orally administered to the rats. The test sample was used in an amount of 1.35 g/kg, calculated as protein, and was administered, using a catheter, as dissolved in 10 ml of an ion exchange water. Before and 30 minutes, 60 minutes, 120 minutes, 180 minutes, 240 minutes and 360 minutes after the administration, the blood of each rat was collected from descending aorta while the rat was narcotized with ether. Upon completion of such collection, the collected blood was subjected to centrifugation and the resulting plasma was preserved at - 80° C. Further, before and 30 minutes, 60 minutes, 120 minutes and 180 minutes after the administration, the right leg soleus muscle of the rat was delivered, was freezed using a clamp freezed with liquid nitrogen and was preserved at - 80° C.

A 200 µl-vol. of plasma and the same volume of 6% sulfosalicylic acid were mixed together, the resulting mixture was centrifuged at 3000 r.p.m. for 10 minutes and the supernatant was freezed for 24 hours. The freezed supernatant was thawed out and centrifuged again. The resulting supernatant was used as a test sample for amino acid analysis. The color-developing reagent used was "Ninhydrin reagent L-8500 Set" manufactured by Wako pure chemical Ltd. The eluting reagent used was "MCI BUFFER L-8500-PF Kit" manufactured by Mitsubishi Chemical Industries Limited. The apparatus for amino acid analysis was a highspeed amino acid analyser (model: L-8500, product of Hitachi, Ltd.) and was used by charging the column (4.6 mm I.D. x 60 mm) with an ion exchange resin (trade name: Hitachi Custom Ion Exchange Resin #2622SC).

Fig. 2 shows the results with regard to amino acids in blood, and Fig. 3 shows the results relating to the free amino acids in flesh.

Figs. 2 and 3 show that the use of the oligopeptide mixture of the present invention improves the amino acid pattern, BCAA/AAA ratio and the like. Further, it was revealed that the drinks, granules, powders, tablets and effervescent compositions of the invention are useful as preparations for compensating the decrease in the ratio of BCAA/AAA caused by the decline of physical strength after exercise.

Table 15

| (g / 100 g) | |
|---|---|
| Asp | 9.3 |
| Thr | 7.6 |
| Ser | 5.5 |
| Glu | 19.9 |
| Gly | 1.4 |
| Ala | 4.5 |
| Val | 3.7 |
| Cys | 2.5 |
| Met | 0.6 |
| Ile | 4.5 |
| Leu | 3.7 |
| Tyr | 0.2 |
| Phe | 0.2 |
| Trp | 0.0 |
| Lys | 10.0 |
| His | 1.2 |
| Arg | 1.9 |
| Pro | 7.9 |
| BCAA (g/100 g) | 12.0 |
| E / N ratio | 0.54 |
| Protein (%) | 75.9 |
| Ash (%) | - |
| Carbohydrate (%) | - |
| Lipid (%) | - |

Test Example II

Model rats suffering from chronic liver failure (A group, 25 rats) were obtained by subjecting 6-week old male Sprague-Dawley rats to portacaval shunt operation (PCS operation). Further, other 6-week old male Sprague-Dawley rats were each subjected to sham operation (B group, 25 rats). All the rats were fed for 2 weeks.

By the following method, these rats were checked for the variations in the concentration of free amino acids in plasma and the concentration of free amino acids in brain owing to a single administration of the oligopeptide mixture. Table 16 below shows the composition and the like of the oligopeptide mixture administered.

After feeding the rats of each group, 5 rats selected from each of A and B groups, respectively were sacrificed. The oligopeptide mixture was administered to the rats remained in each group at a time in an amount of 1.35 g/kg. Fifteen minutes, 30 minutes, 60 minutes and 120 minutes after the administration, 5 rats of the respective groups were sacrificed. The concentration of free amino acid in plasma and the concentration of total free amino acid in brain were determined by the same procedure as in Test Example I. Figs. 4 and 5 show the results.

As seen from Figs. 4 and 5, when the oligopeptide mixture used in the present invention was administered to the rats of the groups A and B, the rats suffering from chronic liver failure exhibited a slightly lower ratio of BCAA/AAA in plasma and brain as compared with those subjected to the sham operation. However, there was found no large difference in the test results between these two kinds of rats.

Therefore it was revealed that the composition of the present invention containing this oligopeptide mixture was useful as a nutritive composition for the patients suffering from chronic liver failure.

Table 16

| (g / 100 g) | |
|---|---|
| Arg | 4.78 |
| Lys | 8.58 |
| His | 1.48 |
| Phe | 0.51 |
| Tyr | 0.34 |
| Leu | 8.91 |
| Ile | 4.78 |
| Met | 0.67 |
| Val | 6.30 |
| Ala | 5.19 |
| Gly | 3.70 |
| Pro | 4.70 |
| Glu | 21.86 |
| Ser | 6.11 |
| Thr | 4.82 |
| Asp | 13.93 |
| Trp | 0.03 |
| Cys | 0.79 |
| BCAA (g/100 g) | 19.99 |
| Molar ratio of BACC/AAA | 31.98 |
| Water (%) | 5.8 |
| Crude protein (%) | 73.2 |
| (Amino nitrogen) | 5.9 |
| Ash (%) | 10.1 |
| Others (%) | 10.9 |

Test Example III

While a 0.05% aqueous solution of sodium phenobarbital was fed to 6-week old male Sprague-Dawley rats as a drink, a 50% solution of carbon tetrachloride in olive oil was subcutaneously administered to the collumn of each rat in an amount of 0.1 ml/100 g body weight two times a week over a period of 12 weeks, giving rats suffering from livercirrhosis.

The rats suffering from livercirrhosis were fed for 3 weeks while the nutritive composition for patients of liver disease according to the invention was given to the rats. Thereafter the rats were compared with those fed with a comparative composition containing casein only as protein source in terms of the concentration of free amino acid in plasma and the concentration of ammonia in blood. Table 17 shows the composition and the like of the oligopeptide mixture used. Table 18 shows the compositions of the nutritive composition for patients of liver disease according to the invention and the comparative composition used.

The concentration of free amino acid in plasma was determined in the same manner as in Test Example I. The concentration of ammonia in blood was determined with use of an apparatus, "Ammonia-Test Wako", manufactured by Wako pure chemical Ltd.

After feeding the rats suffering from livercirrhosis with the nutritive composition for patients of liver disease according to the invention or with the comparative composition for 3 weeks, the rats were checked for the concentration of free amino acid in plasma and the concentration of ammonia in blood with the results shown in Fig. 6.

As clear from the results, the rats fed with the composition of the invention were significantly decreased in the concentration of AAA in plasma and significantly increased in the molar ratio of BCAA/AAA. Further, the concentration of ammonia in blood was significantly kept low as compared with those fed with the comparative composition.

The above results indicate that the nutritive composition for patients of liver disease according to the invention containing the oligopeptide mixture improves the imbalanced pattern of amino acids in blood and the increase in the concentration of ammonia in blood both of which were found in patients of liver disease, accelerates the treatment and prevention of hepatogeneous encephalopathia and is useful for improving the nutriture of the patients.

Table 17

| Composition of amino acid (wt.%) | |
|---|---|
| Asp | 14.3 |
| Thr | 5.0 |
| Ser | 6.3 |
| Glu | 23.7 |
| Gly | 3.8 |
| Ala | 5.1 |
| Val | 6.1 |
| Cys | 1.1 |
| Met | 0.7 |
| Ile | 4.8 |
| Leu | 8.6 |
| Tyr | 0.3 |
| Phe | 0.5 |
| Lys | 8.2 |
| His | 1.2 |
| Arg | 5.3 |
| Pro | 4.9 |
| Water (%) | 6.7 |
| Crude protein (%) | 75.5 |
| Ash (%) | 9.7 |

Table 18

| | Composition of Invention | Comparative composition |
|---|---|---|
| Oligopeptide mixture | 6.6% | - |
| Casein | 5.6% | 11.1% |
| Corn starch | 64% | 64% |
| Sucrose | 10% | 10% |
| Corn oil | 5% | 5% |
| Vitamin | 1% | 1% |
| Mineral | 5% | 5% |
| Cellulose | 2.8% | 3.9% |

## Claims

1. An oligopeptide mixture prepared by the hydrolysis of an animal or plant protein with enzymes, the oligopeptide mixture being characterized in that the mixture has an average molecular weight of 200 to 5000, and contains not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7.

2. An oligopeptide mixture according to claim 1 wherein the oligopeptide mixture has an average molecular weight of 200 to 3000 and contains not more than 30% by weight of free amino acids and, based on the total amino acid composition, 17 to 25% by weight of a branched-chain amino acid, 0.1 to 2.0% by weight of an aromatic amino acid and 0.5 to 1.2% by weight of a methionine, the (branched-chain amino acid/aromatic amino acid) molar ratio being not less than 10.

3. A process for preparing the oligopeptide mixture as defined in claim 1, the process being characterized by comprising the steps of subjecting an animal or plant protein to enzymolysis with an endoprotease and an exoprotease at neutrality range in the presence of an aqueous medium, treating the resulting decomposition product with an adsorbent, and when required, concentrating and/or drying the product.

4. A nutritive composition for patients of liver diseases, the composition being characteried by containing an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight cf a branched-chain amino acid, 0.1 to 2.5% by weight of an aromatic amino acid and 0.5 to 1.5% by weight of a methionine, the (branched-chain amino acid/aromatic amino acid) molar ratio being not less than 7.

5. A nutritive composition according to claim 4 wherein the oligopeptide mixture has an average molecular weight of 200 to 3000 and contains not more than 30% by weight of free amino acids and, based on the total amino acid composition, 17 to 25% by weight of a branched-chain amino acid, 0.1 to 2.0% by weight of an aromatic amino acid and 0.5 to 1.2% by weight of a methionine, the (branched-chain amino acid/aromatic amino acid) molar ratio being not less than 10.

6. A nutritive composition for patients of liver diseases, the composition being characterized in that the composition contains 5 to 70% by weight, in terms of the dry weight, of an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7, and that the composition contains 10 to 90% by weight of total nitrogen compound as a protein source, 10 to 80% by weight of carbohydrate and 0 to 15% by weight of lipid in terms of the dry weight.

**7.** A nutritive composition according to claim 6 wherein the oligopeptide mixture has an average molecular weight of 200 to 3000 and contains not more than 30% by weight of free amino acids and, based on the total amino acid composition, 17 to 25% by weight of a branched-chain amino acid, 0.1 to 2.0% by weight of an aromatic amino acid and 0.5 to 1.2% by weight of a methionine, the (branched-chain amino acid/aromatic amino acid) molar ratio being not less than 10.

**8.** A nutritive composition according to claim 6 or 7 wherein the composition contains 5 to 20% by weight of the oligopeptide mixture in terms of the dry weight, and wherein the composition contains 15 to 40% by weight of total nitrogen compound as a protein source, 50 to 70% by weight of carbohydrate and 3 to 10% by weight of lipid in terms of the dry weight.

**9.** A nutritive or food composition characterized in that it contains an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight of a branched-chain amino acid and 0.1 to 2.5% by weight of an aromatic amino acid, the (branched-chain amino acid/aromatic amino acid) molar ratio being not less than 7.

**10.** A nutritive or food composition characterized in that it contains an oligopeptide mixture prepared by the hydrolysis of an animal or plant protein with enzymes, the mixture having an average molecular weight of 200 to 5000 and containing not more than 35% by weight of free amino acids and, based on the total amino acid composition, 15 to 25% by weight of a branched-chain amino acid, 0.1 to 2.5% by weight of an aromatic amino acid and 0.5 to 1.5% by weight of a methionine, the (branched-chain amino acid/aromatic amino acid) molar ratio being not less than 7.

**11.** A nutritive or food composition according to claim 9 or 10 which is in the form of a beverage having the oligopeptide mixture as the essential component dissolved in water.

**12.** A nutritive or food composition according to claim 9 or 10 which is in the form of granules containing the oligopeptide mixture as the essential component.

**13.** A nutritive or food composition according to claim 9 or 10 which is in the form of powder containing the oligopeptide mixture as the essential component.

**14.** A nutritive or food composition according to claim 9 or 10 which is in the form of tablets containing the oligopeptide mixture as the essential component.

**15.** A nutritive or food composition according to claim 9 or 10 which is an effervescent preparation in the form of granules, powder or tablets containing the oligopeptide mixture, sodium hydrogencarbonate and/or sodium carbonate and a neutralizing agent as the essential components.

FIG. 1

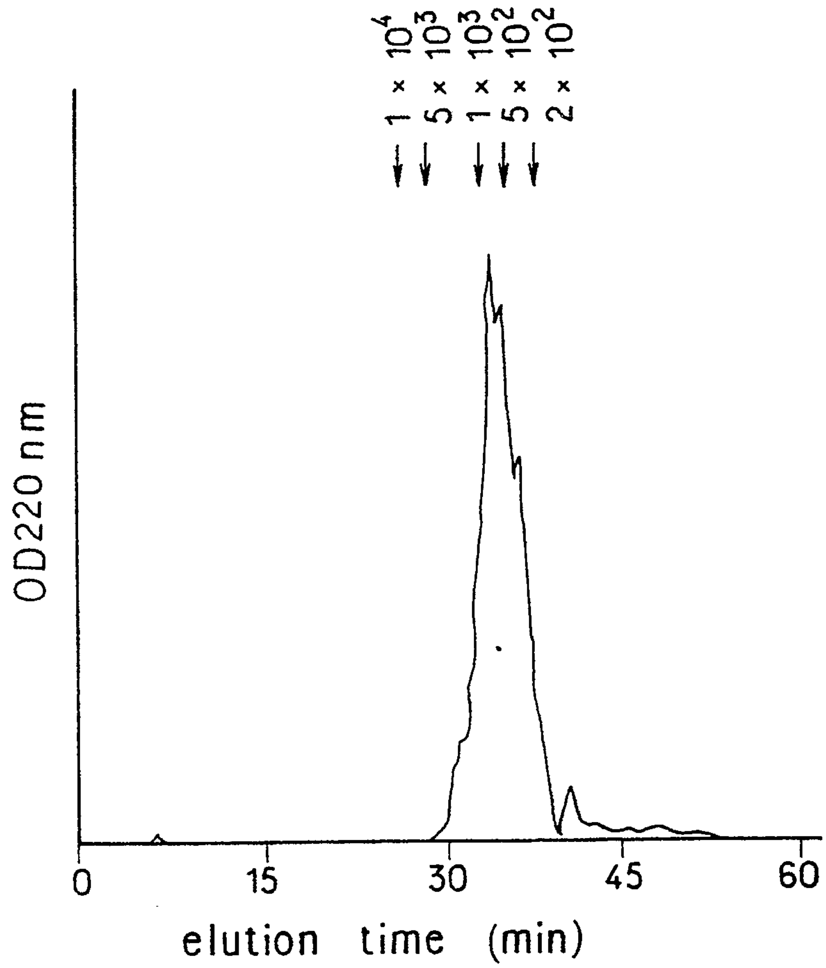
1 × 10^4
5 × 10^3
1 × 10^3
5 × 10^2
2 × 10^2
OD220 nm
0
15
30
45
60
elution time (min)

FIG. 2

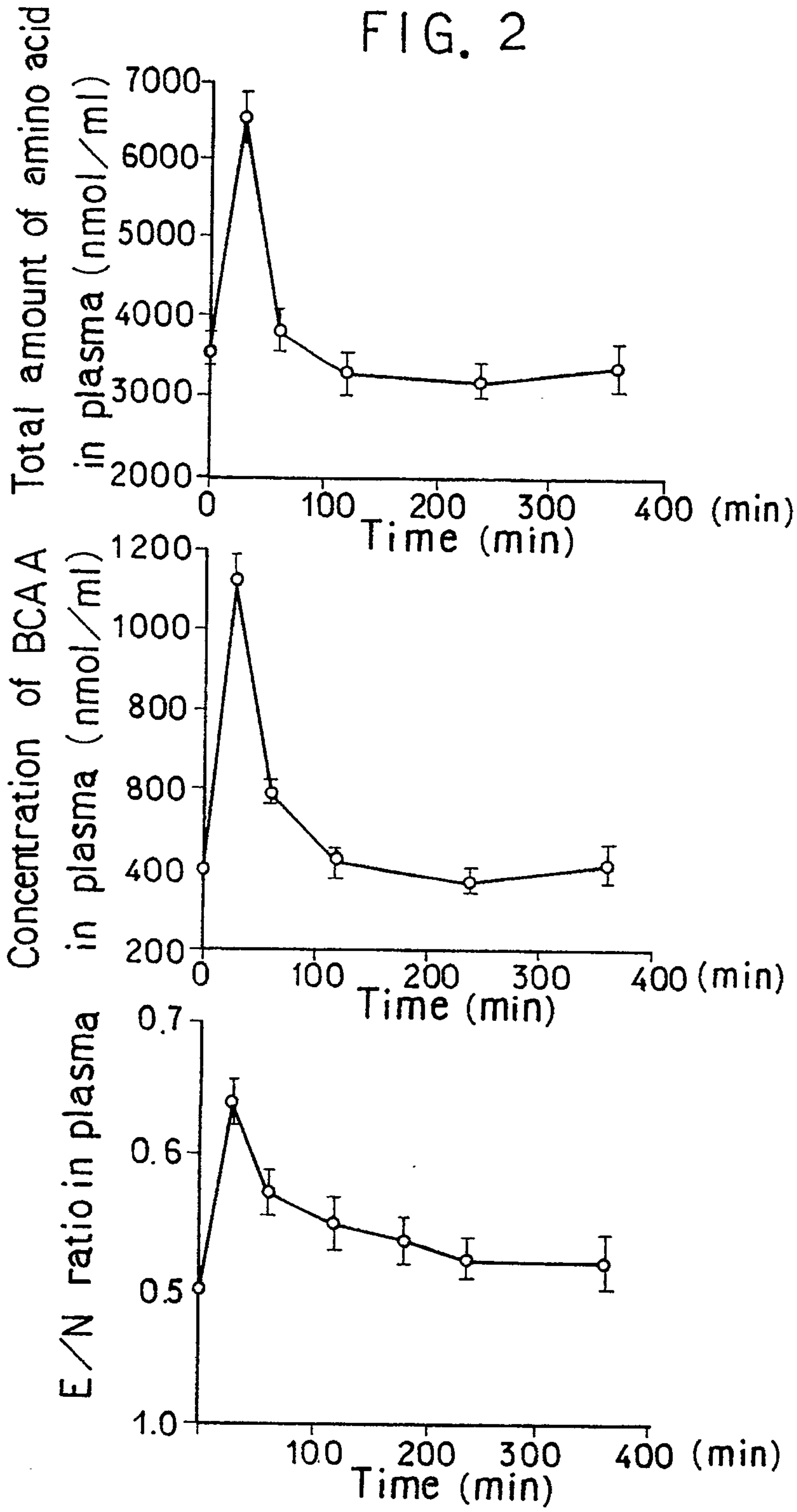
Total amount of amino acid in plasma (nmol/ml)
7000
6000
5000
4000
3000
2000
0
100
200
300
400 (min)
Time (min)
Concentration of BCAA in plasma (nmol/ml)
1200
1000
800
800
400
200
0
100
200
300
400 (min)
Time (min)
E/N ratio in plasma
0.7
0.6
0.5
1.0
100
200
300
400 (min)
Time (min)

FIG. 3

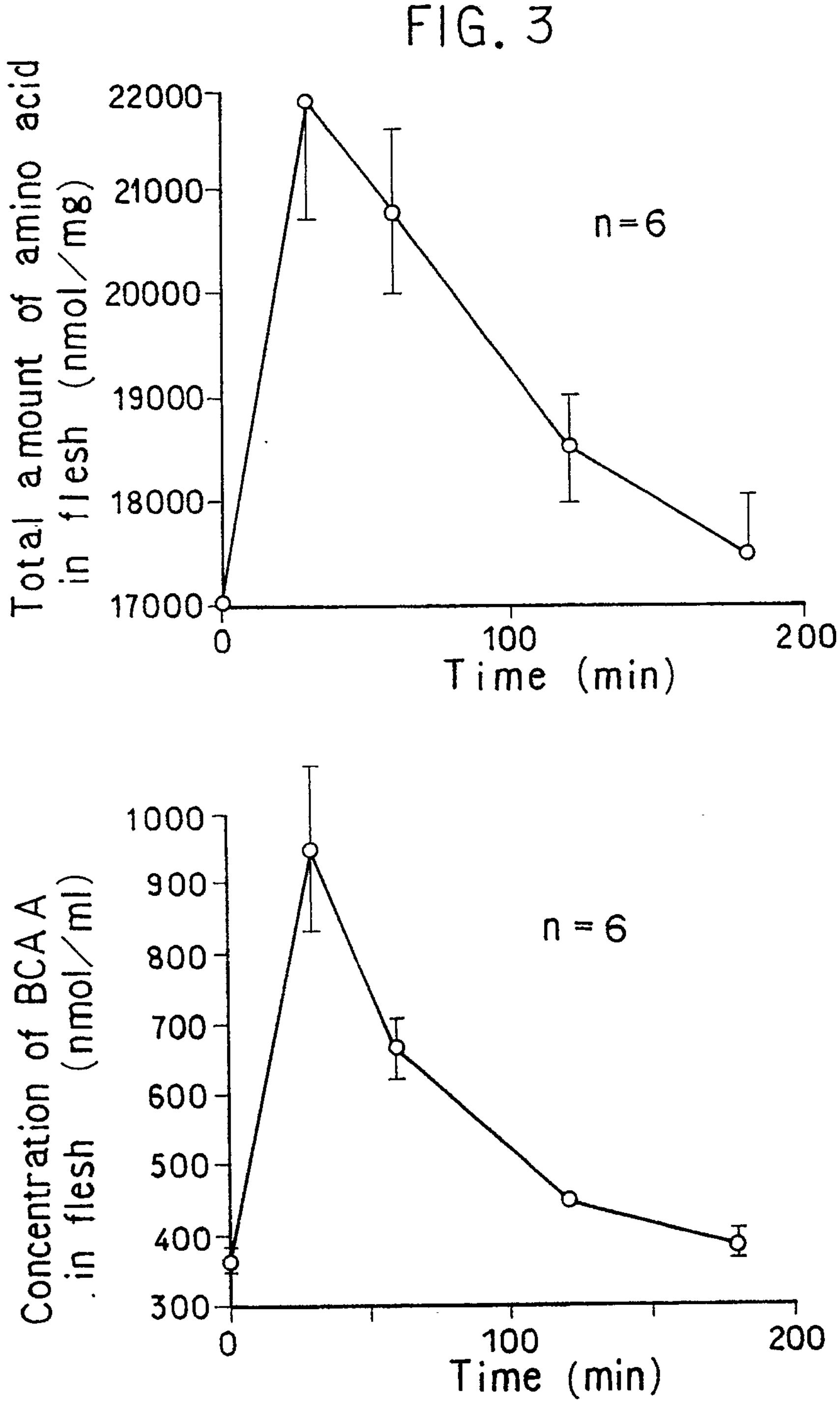
Total amount of amino acid in flesh (nmol/mg)
22000
21000
20000
19000
18000
17000
n=6
0
100
200
Time (min)
Concentration of BCAA in flesh (nmol/ml)
1000
900
800
700
600
500
400
300
n = 6
0
100
200
Time (min)

FIG. 4

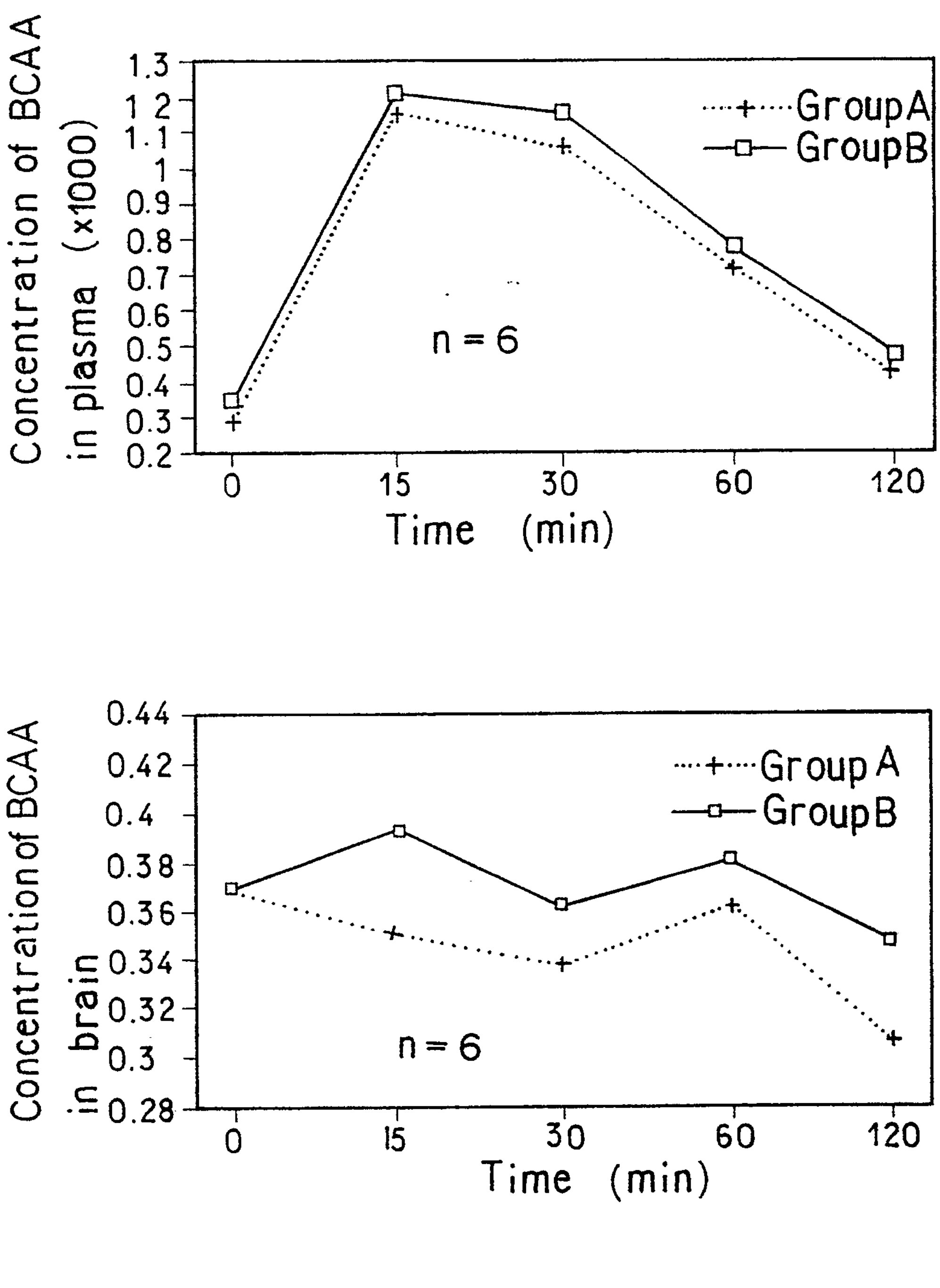
Concentration of BCAA in plasma (x1000)
1.3
1.2
1.1
1
0.9
0.8
0.7
0.6
0.5
0.4
0.3
0.2
GroupA
GroupB
n = 6
0
15
30
60
120
Time (min)
Concentration of BCAA in brain
0.44
0.42
0.4
0.38
0.36
0.34
0.32
0.3
0.28
Group A
GroupB
n = 6
0
15
30
60
120
Time (min)

FIG. 5

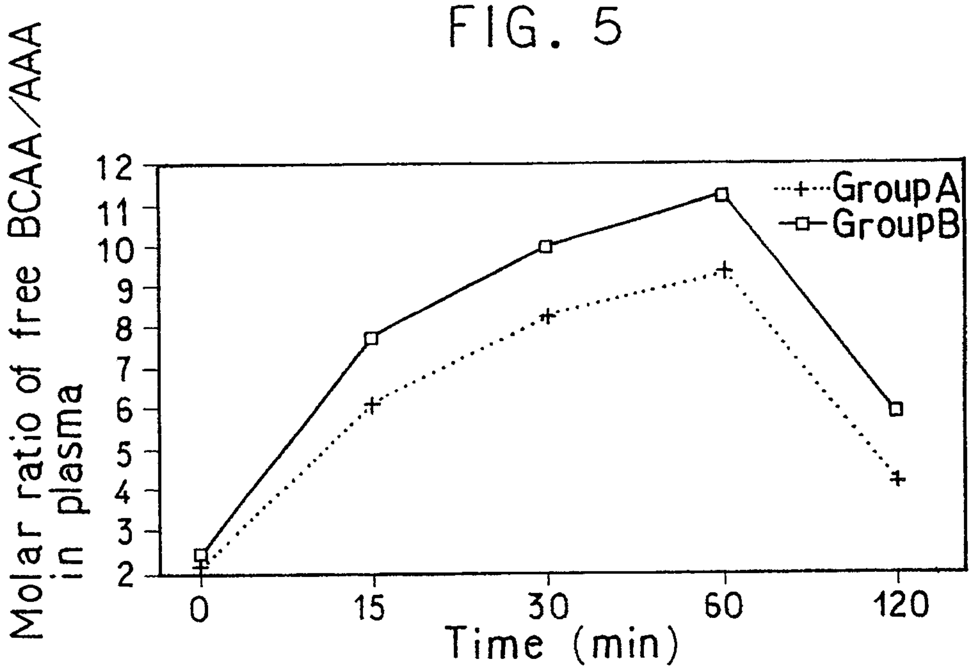
Molar ratio of free BCAA/AAA in plasma
12
11
10
9
8
7
6
5
4
3
2
GroupA
GroupB
0
15
30
60
120
Time (min)

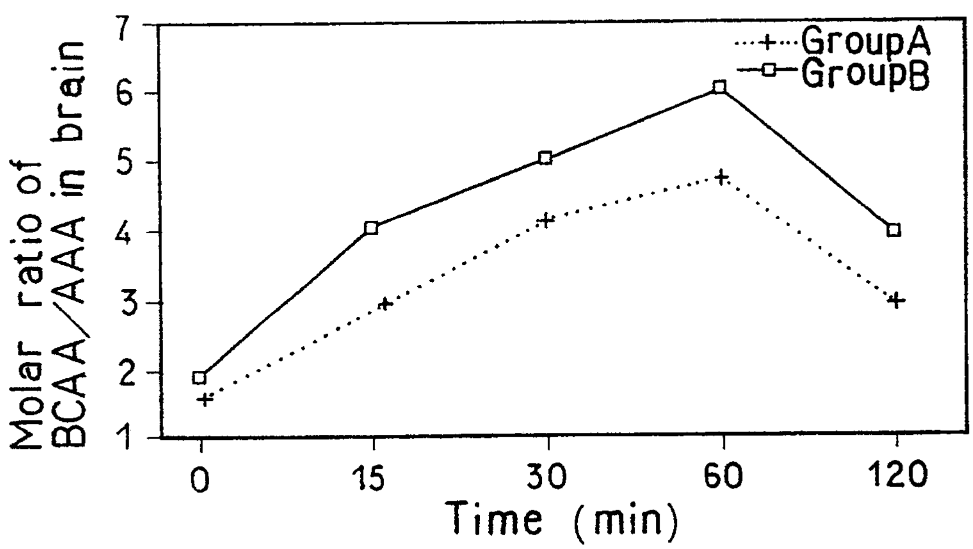
Molar ratio of BCAA/AAA in brain
7
6
5
4
3
2
1
GroupA
GroupB
0
15
30
60
120
Time (min)

FIG. 6

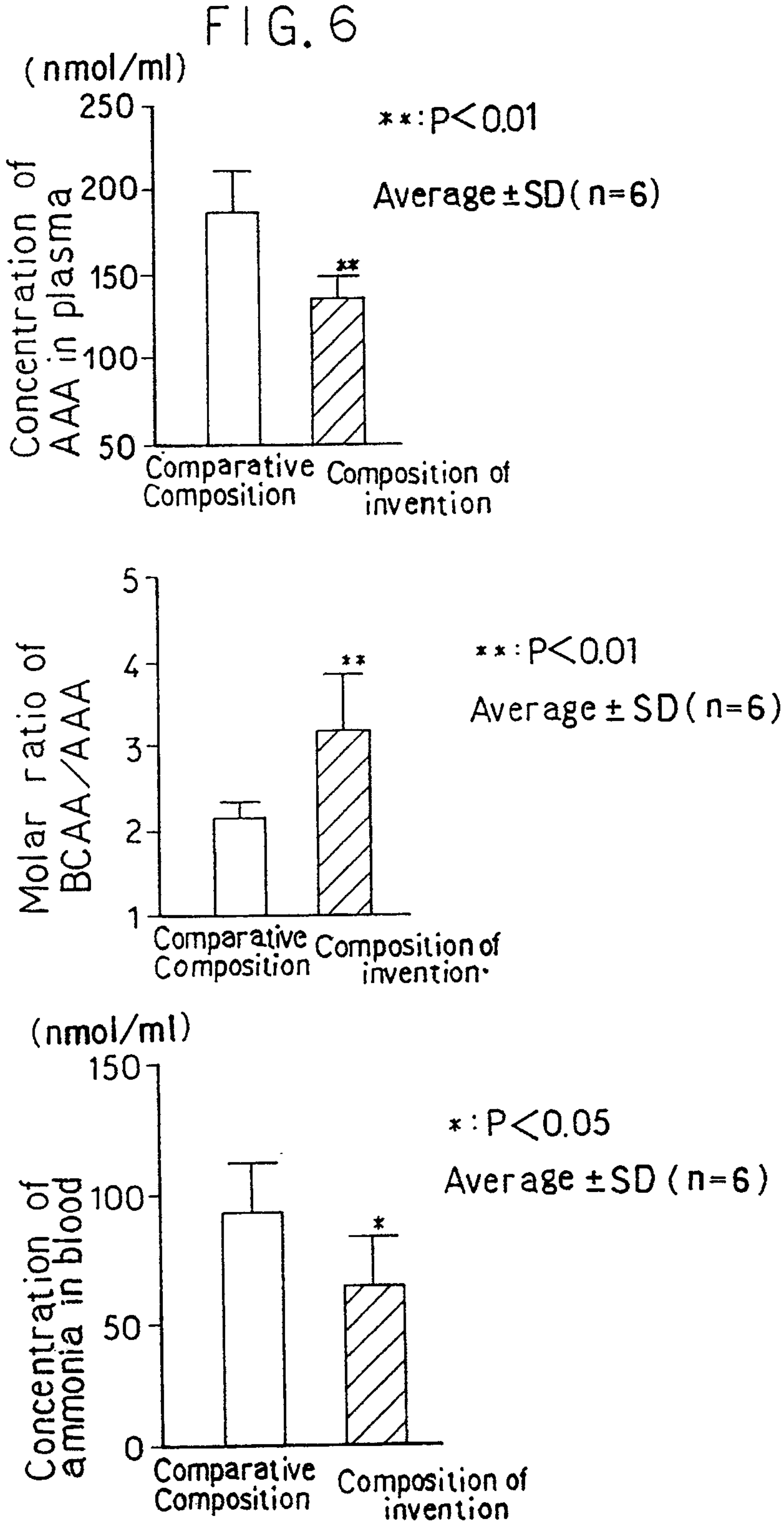
(nmol/ml)
250
200
150
100
50
Concentration of AAA in plasma
**: P<0.01
Average ± SD (n=6)
**
Comparative Composition
Composition of invention
5
4
3
2
1
Molar ratio of BCAA/AAA
**: P<0.01
Average ± SD (n=6)
**
Comparative Composition
Composition of invention
(nmol/ml)
150
100
50
0
Concentration of ammonia in blood
*: P<0.05
Average ± SD (n=6)
*
Comparative Composition
Composition of invention

# INTERNATIONAL SEARCH REPORT

International Application No PCT/JP90/00598

**I. CLASSIFICATION OF SUBJECT MATTER** (if several classification symbols apply, indicate all) [6]

According to International Patent Classification (IPC) or to both National Classification and IPC

Int. $Cl^5$ A23L1/305, 1/48, C12P21/06

**II. FIELDS SEARCHED**

Minimum Documentation Searched [7]

| Classification System | Classification Symbols |
|---|---|
| IPC | A23L1/305, 1/48, C12P21/06, A61K37/18 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, A, 61-68426 (Fuji Oil Co., Ltd.), 8 April 1986 (08. 04. 86), (Family: none) | 1 - 15 |
| A | JP, B2, 62-61039 (Institut National de la Recherche Agronomique), 18 December 1987 (18. 12. 87) & EP, A1, 22019 | 1 - 15 |
| A | JP, A, 51-5364 (Massachuset General Hospital), 19 October 1976 (19. 10. 76), (Family: none) | 1 - 15 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 27, 1990 (27. 07. 90) | August 6, 1990 (06. 08. 90) |
| **International Searching Authority** | **Signature of Authorized Officer** |
| Japanese Patent Office | |